# EUROPEAN PATENT SPECIFICATION

(11) **EP 1 942 107 B1**
(45) Date of publication and mention of the grant of the patent: **05.03.2014**
(21) Application number: 06797233.1
(22) Date of filing: 31.08.2006
(51) Int. Cl.: C07D 401/14, C09K 11/06, H01L 51/50

(54) **COMPOUND HAVING TRIAZOLE RING STRUCTURE SUBSTITUTED WITH PYRIDYL GROUP AND ORGANIC ELECTROLUMINESCENT DEVICE**
VERBINDUNG MIT EINER DURCH EINE PYRIDYLGRUPPE SUBSTITUIERTEN TRIAZOL-RINGSTRUKTUR UND ORGANISCHES ELEKTROLUMINESZENTES GERÄT
COMPOSÉ AYANT UNE STRUCTURE DE CYCLE TRIAZOLE AVEC UN GROUPE PYRIDYLE SUBSTITUÉ ET DISPOSITIF ÉLECTROLUMINESCENT ORGANIQUE

(30) Priority: 31.08.2005 JP 2005250734
(43) Date of publication of application: 09.07.2008
(73) Proprietor: Hodogaya Chemical Co., Ltd., Tokyo 104-0028 (JP); Shinshu University, Matsumoto-shi, Nagano 390-8621 (JP)
(72) Inventor: MIKI, Tetsuzo, Tokyo 104-0028 (JP); YOKOYAMA, Norimasa, Tokyo 104-0028 (JP); TANIGUCHI, Yoshio, Nagano 386-0018 (JP); ICHIKAWA, Musubu, Nagano 386-0018 (JP)
(74) Representative: HOFFMANN EITLE
(86) International application number: PCT/JP2006/317273
(87) International publication number: WO 2007/026847

(56) References cited:
- WO-A1-2005/092888
- JP-A- 07 041 759
- JP-A- 2000 100 570
- ICHIKAWA M. ET AL.: 'New triazole derivatives as hole-blocking and electron-transporting materials for organic light-emitting devices' DIGEST OF TECHNICAL PAPERS - SOCIETY FOR INFORMATION DISPLAY INTERNATIONAL SYMPOSIUM vol. 37, no. BK. 1, 2006, pages 45 - 48, XP008072943
- MANDAL S.K.: 'Synthesis, structure and electrochemistry of a novel dinuclear cobalt (II) complex containing pyrrole units: an unusual reductive electropolymerization of the dinuclear cobalt (II) complex' INORGANICA CHIMICA ACTA vol. 209, no. 1, 1993, pages 1 - 4, XP003003280

## Description

### TECHNICAL FIELD

This invention relates to a compound suitable for an organic electroluminescence (EL) device which is a self-luminescent device suitable for various display apparatuses and to a device. More specifically, the invention relates to a compound having a triazole ring structure to which a substituted pyridyl group is bonded and to an organic EL device using the compound.

### BACKGROUND ART

Since the organic EL devices are self-luminescent devices, they are bright, excellent in visibility, and capable of giving clear display as compared to liquid crystal devices, and studies thereon have actively been conducted.

In 1987, C. W. Tang et al. of Eastman Kodak Co. have turned the organic EL device using an organic material into practical utilization by developing a stacked structure device wherein various functions are respectively distributed to materials. They stack a fluorescent material capable of transporting electrons and an organic substance capable of transporting holes, and injected both of the charges into the fluorescent material layer to emit a light, thereby achieving a high luminance of 1,000 cd/m² or more at a voltage of 10 V or less (see Patent Document 1 and Patent Document 2, for example).

Patent Document 1: JP-A-8-48656
Patent Document 2: Japanese Patent No. 3194657

Many improvements have been made up to the present for putting organic EL devices into practical use, and high efficiency and durability have been achieved by an electroluminescence device wherein an anode, a hole injection layer, a hole transport layer, a luminescent layer, an electron transport layer, an electron injection layer, and a cathode are provided in this order on a substrate, thereby further segmentalizing various roles (see Non-Patent Document 1, for example).

Non-Patent Document 1: Preprints for 9th Workshop of Japan Applied Physics, pages 55 to 61, (2001)

Also, there has been an attempt for utilizing a triplet exciton in the aim of a further improvement in luminescent efficiency, and use of phosphorescent material has been studied (see Non-Patent Document 2, for example).

Non-Patent Document 2: Preprints for 9th Workshop of Japan Applied Physics, pages 23 to 31, (2001)

The luminescent layer may be produced by doping a compound having electron transport property, which is generally called a host material, with a fluorescent material or phosphorescent material. As disclosed in the above-mentioned workshop preprints, selection of an organic material in an organic EL device greatly influences on various properties of the device, such as efficiency and durability.

In organic EL devices, luminescence is obtained when charges injected from the electrodes are re-combined in the luminescent layer. Since the mobility of holes is higher than that of electrons, a problem of reduction in efficiency caused by a part of holes passing through the luminescent layer arises. Therefore, an electron transport material having high hole blocking property has been demanded.

Although tris(8-hydroxyquinoline)aluminum (hereinafter abbreviated as Alq) which is a typical luminous material is generally used as an electron transport material, Alq is not considered to have hole blocking property.

Therefore, as an electron transport material having excellent hole blocking property, 3-(4-biphenylyl)-4-phenyl-5-(4-t-butylphenyl)-1,2,4-triazole (hereinafter abbreviated as TAZ) has been proposed (see Patent Documents 3 and 4, for example).

Patent Document 3: Japanese Patent No. 2734341
Patent Document 4: Japanese Patent No. 07041759.

Since TAZ has a work function as large as 5.8 eV and a high hole blocking property, this compound has been used as a hole blocking layer having electron transport property to be stacked on a cathode side of a fluorescent luminescent layer or a phosphorescent luminescent layer formed by vacuum vapor deposition or coating, thereby contributing to achievement of high efficiency of organic EL devices (see Non-Patent Document 3, for example).

Non-Patent Document 3: 28p-A-6 Preprints for 50th Lecture of Association of Japan Applied Physics, page 1413, (2003)

However, TAZ involves a great problem of having a low electron transport property. Accordingly, it has been necessary to combine it with an electron transport material having higher electron transport property in producing organic EL devices (see Non-Patent Document 4, for example).

Non-Patent Document 4: Journal of Organic Molecular Electronics and Bioelectronics of Japan Society of Applied Physics, Vol. 11, No. 1, pages 13 to 19, (2000)

An electron transport material having improved electron transport property and high hole blocking property as well as an organic EL device having high efficiency have been demanded.

### DISCLOSURE OF THE INVENTION

### PROBLEMS TO BE SOLVED BY THE INVENTION

An object of this invention is to provide an organic compound excellent in electron transport property and hole blocking property as a material for highly efficient organic EL device, and also provide a highly efficient organic EL device by using the compound. Examples of the physical properties of an organic compound suitable for this invention include (1) high electron mobility and (2) excellent hole blocking property. Also, examples of the physical properties of a device suitable for this invention include (1) low driving voltage and (2) high luminescent efficiency.

### MEANS TO SOLVE THE PROBLEMS

In order to attain the above objects, the inventors of this invention have noted the fact that a pyridine ring is electron attractive and have designed and chemically synthesized novel organic compounds in which a substituted pyridine ring.is bonded to a triazole ring. The inventors have produced various organic EL devices by using the compounds and have conducted extensive characteristics evaluations of the devices to accomplish this invention.

That is, this invention relates to a compound represented by the general formula (1) having a triazole ring structure to which a substituted pyridyl group is bonded, and also relates to an organic electroluminescence device comprising a pair of electrodes and at least one organic layer sandwiched between the electrodes, wherein the compound is used as a constituting material of the at least one organic layer:

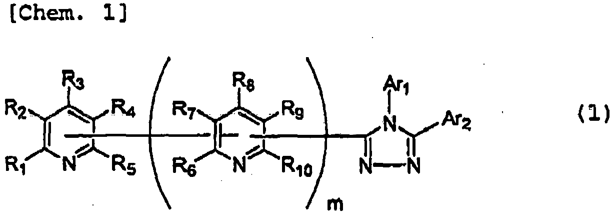

(wherein Ar₁ and Ar₂ may be the same or different and each represents a substituted or unsubstituted aromatic hydrocarbon group, a substituted or unsubstituted aromatic heterocyclic group, or a substituted or unsubstituted condensed polycyclic aromatic group; any one of R₁, R₂, R₃, R₄, and R₅ is a bonding group and the rest thereof may be the same or different and each represents a hydrogen atom, a fluorine atom, a cyano group, an alkyl group, a substituted or unsubstituted aromatic hydrocarbon group, a substituted or unsubstituted aromatic heterocyclic group, or a substituted or unsubstituted condensed polycyclic aromatic group; any two of R₆, R₇, R₈, R₉, and R₁₀ are bonding groups and the rest thereof may be the same or different and each represents a hydrogen atom, a fluorine atom, a cyano group, an alkyl group, a substituted or unsubstituted aromatic hydrocarbon group, a substituted or unsubstituted aromatic heterocyclic group, a substituted or unsubstituted condensed polycyclic aromatic group; and m represents an integer of 1 to 4).

Specific examples of the aromatic hydrocarbon group, the aromatic heterocyclic group, or the condensed polycyclic aromatic group in the substituted or unsubstituted aromatic hydrocarbon group, the substituted or unsubstituted aromatic heterocyclic group, or the substituted or unsubstituted condensed polycyclic aromatic group represented by Ar₁ and Ar₂ in the general formula (1) include a phenyl group, a biphenyl group, a terphenyl group, a tetrakisphenyl group, a styryl group, a naphthyl group, an anthryl group, an acenaphthenyl group, a fluorenyl group, a phenanthryl group, an indenyl group, a pyrenyl group, a pyridyl group, a pyrimidyl group, a furanyl group, a pyronyl group, a thiophenyl group, a quinolyl group, a benzofuranyl group, a benzothiophenyl group, an indolyl group, a carbazolyl group, a benzoxazolyl group, a quinoxalyl group, a benzoimidazolyl group, a pyrazolyl group, a dibenzofuranyl group, and a dibenzothiophenyl group.

Specific examples of the substituent group in the substituted or unsubstituted aromatic hydrocarbon group, the substituted or unsubstituted aromatic heterocyclic group, or the substituted or unsubstituted condensed polycyclic aromatic group represented by Ar₁ and Ar₂ in the general formula (1) include a fluorine atom, a chlorine atom, a cyano group, a hydroxide group, a nitro group, an alkyl group, an alkoxy group, an amino group, a trifluoromethyl group, a phenyl group, a biphenyl group, a terphenyl group, a naphthyl group, a phenanthryl group, an aralkyl group, a fluorenyl group, an indenyl group, a pyridyl group, a pyrimidyl group, a furanyl group, a pyronyl group, a thiophenyl group, a quinolyl group, a benzofuranyl group, a benzothiophenyl group, an indolyl group, a carbazolyl group, a benzoxazolyl group, a quinoxalyl group, a benzoimidazolyl group, a pyrazolyl group, a dibenzofuranyl group, and a dibenzothiophenyl group, and the substituent group may be further substituted.

Specific examples of the aromatic hydrocarbon group, the aromatic heterocyclic group, or the condensed polycyclic aromatic group in the substituted or unsubstituted aromatic hydrocarbon group, the substituted or unsubstituted aromatic heterocyclic group, or the substituted or unsubstituted condensed polycyclic aromatic group among the substituent groups on the pyridyl group represented by R₁ to R₁₀ in the general formula (1) include a phenyl group, a biphenyl group, a terphenyl group, tetrakisphenyl group, a styryl group, a naphthyl group, an anthryl group, am acenaphthenyl group, a fluorenyl group, a phenanthryl group, an indenyl group, a pyrenyl group, a pyrimidyl group, a furanyl group, a pyronyl group, a thiophenyl group, a quinolyl group, a benzofuranyl group, a benzothiophenyl group, an indolyl group, a carbazolyl group, a benzoxazolyl group, a quinoxalyl group, a benzoimidazolyl group, a pyrazolyl group, a dibenzofuranyl group, and a dibenzothiophenyl group.

Specific examples of the substituent group in the substituted or unsubstituted aromatic hydrocarbon group, the substituted or unsubstituted aromatic heterocyclic group, or the substituted or unsubstituted condensed polycyclic aromatic group among the substituent groups on the pyridyl group represented by R₁ to Rio in the general formula (1) include a fluorine atom, a chlorine atom, a cyano group, a hydroxide group, a nitro group, an alkyl group, an alkoxy group, an amino group, a trifluoromethyl group, a naphthyl group, an aralkyl group, a fluorenyl group, an indenyl group, a pyridyl group, a pyrimidyl group, a furanyl group, a pyronyl group, a thiophenyl group, a quinolyl group, a benzofuranyl group, a benzothiophenyl group, an indolyl group, a carbazolyl group, a benzoxazolyl group, a quinoxalyl group, a benzoimidazolyl group, a pyrazolyl group, a dibenzofuranyl group, and a dibenzothiophenyl group, and the substituent group may be further substituted.

The compound of this invention represented by the general formula (1) and having a triazole ring structure to which a substituted pyridyl group is bonded has a higher electron mobility and more excellent hole blocking property than the conventional material, TAZ.

The compound of this invention represented by the general formula (1) and having a triazole ring structure to which a substituted pyridyl group is bonded is usable as a constituting material of an electron transport layer of an organic EL device. By using the material having a higher electron mobility and more excellent hole blocking property than TAZ as a hole transport layer-cum-electron transport layer, it is possible to achieve effects of prominently reducing a driving voltage, improving luminescent efficiency, and enabling production of a highly efficient and durable organic EL device.

Since the compound of this invention represented by the general formula (1) having a triazole ring structure to which a substituted pyridyl group is bonded has a high hole blocking property, it is possible to use the compound as a constituting material of a hole blocking layer of an organic EL device in combination with another electron transport material.

The compound of this invention represented by the general formula (1) and having a triazole ring structure to which a substituted pyridyl group is bonded may also be used as a constituting material of a luminescent layer of an organic EL device. With the use of a luminescent layer obtained by using the material of this invention excellent in electron transport property and having a wide band gap as compared to TAZ as a host material of the luminescent layer and by causing the compound to carry a fluorescent material or phosphorescent material which is called dopant, it is possible to produce an organic EL device reduced in driving voltage and improved in luminescent efficiency.

Since the organic EL device of this invention is obtained by using the compound having a triazole ring structure to which a substituted pyridyl group is bonded, the compound having a higher electron mobility and more excellent hole blocking property than TAZ, a driving voltage thereof is prominently reduced and luminescent efficiency is improved, thereby realizing high efficiency.

### ADVANTAGE OF THE INVENTION

This invention relates to a compound having a triazole ring structure to which a substituted pyridyl group is bonded, which is useful as a constituting material of an electron transport layer, a hole blocking layer-cum-electron transport layer, a hole blocking layer, or a luminescent layer of an organic EL device, and also relates to the organic EL device produced using the compound. By this invention, it is possible to considerably reduce a driving voltage of an organic EL device using an electron transport material excellent in hole blocking property, thereby realizing high efficiency.

### BRIEF DESCRIPTION OF THE DRAWINGS

Fig. 1 is a chart of the NMR spectrum of BpyTAZ01 (Compound 2).
Fig. 2 is a chart of the NMR spectrum of BpyTAZ02 (Compound 9).
Fig. 3 is a chart of the NMR spectrum of Compound 11.
Fig. 4 is a chart of the NMR spectrum of Compound 15.
Fig. 5 is a chart of the NMR spectrum of Compound 16.
Fig. 6 is a diagram showing the structure of the EL device of Example 8.
Fig. 7 is a diagram showing the structure of the EL device of Example 10.

### BEST MODE FOR CARRYING OUT THE INVENTION

The compound of this invention having a triazole ring structure to which a substituted pyridyl group is bonded is a novel compound, and.it is possible to synthesize the compound having a triazole ring structure to which a substituted pyridyl group is bonded by a cyclization reaction of corresponding diacylhydrazine with arylamine in the presence of phosphorus trichloride.

Also, in the aromatic hydrocarbon group, the aromatic heterocyclic group, or the condensed polycyclic aromatic group represented by Ar₂ of the general formula (1), it is possible to further introduce a substituted or unsubstituted aromatic hydrocarbon group, substituted or unsubstituted aromatic heterocyclic group, or substituted or unsubstituted condensed polycyclic aromatic group by a cross coupling reaction such as the Suzuki coupling of a compound wherein at least one hydrogen is substituted by a halogen atom with arylboronic acid.

Specific examples of preferred compounds among the compounds represented by the general formula (1) having a triazole structure to which a substituted pyridyl group is bonded are shown below, but this invention is not limited to the compounds.

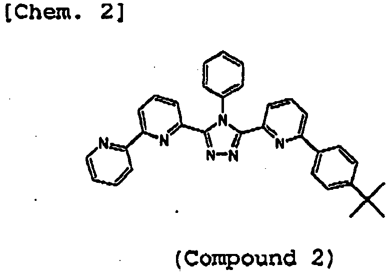

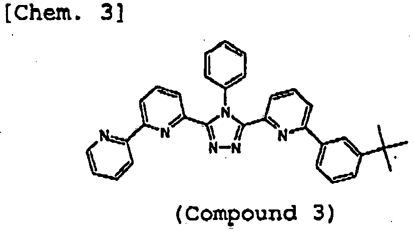

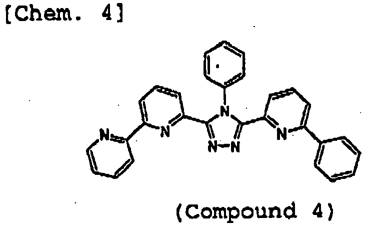

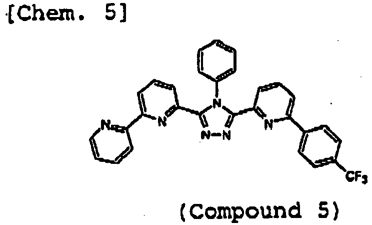

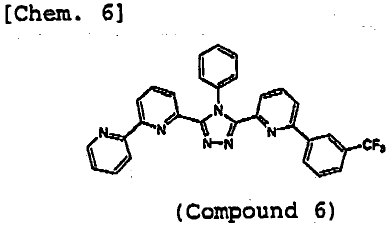

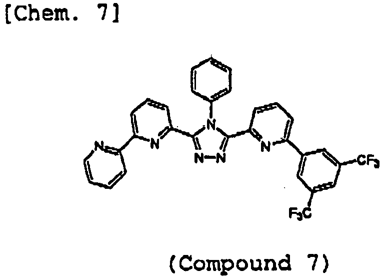

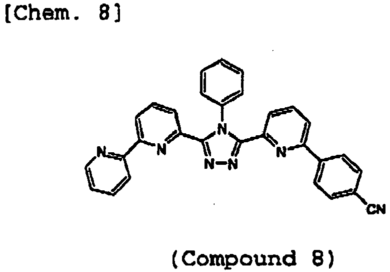

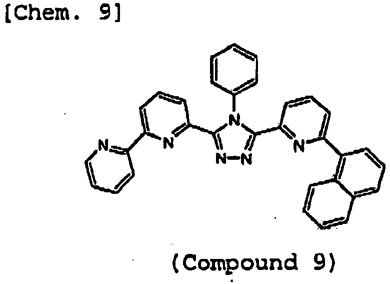

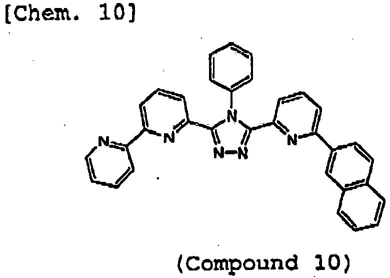

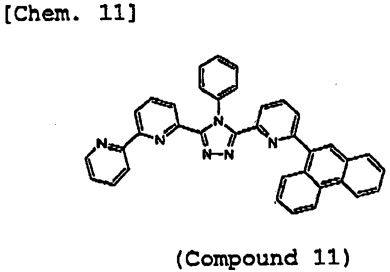

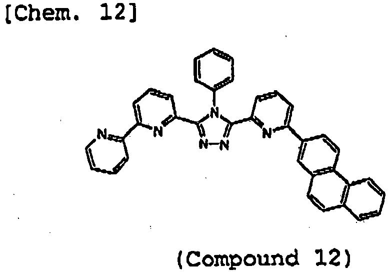

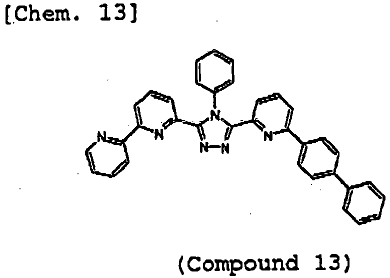

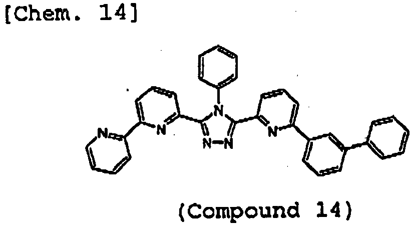

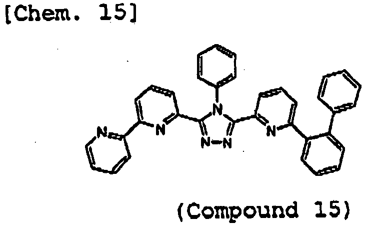

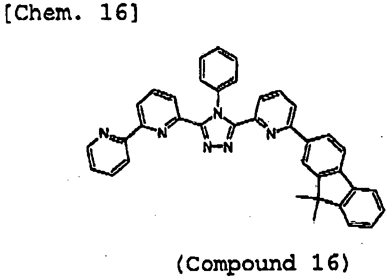

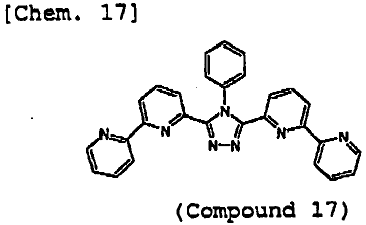

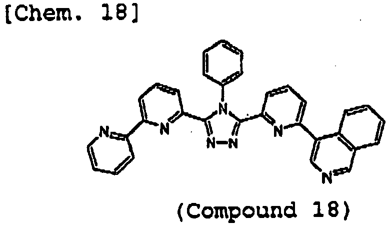

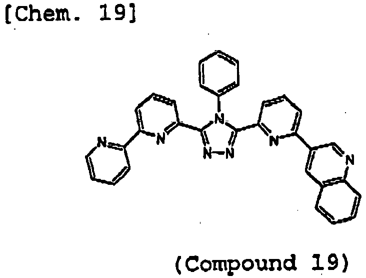

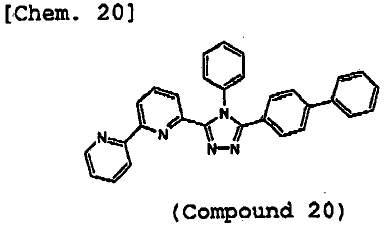

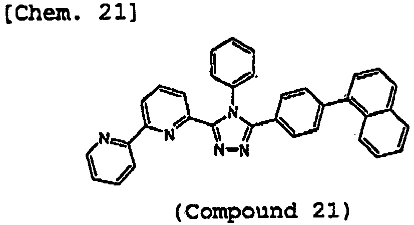

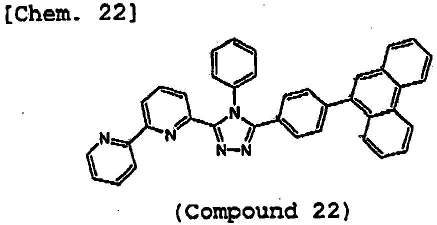

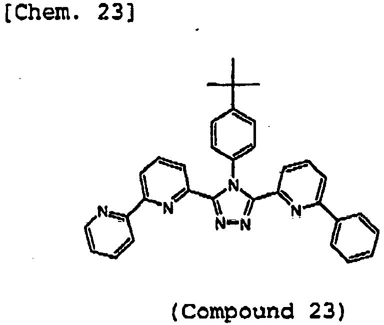

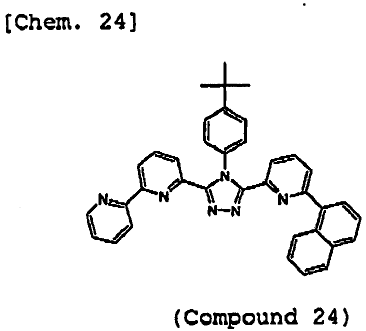

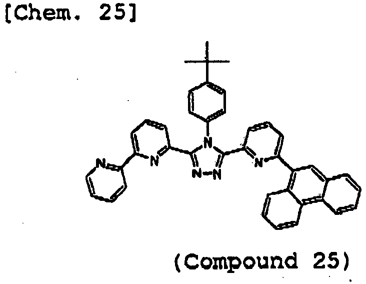

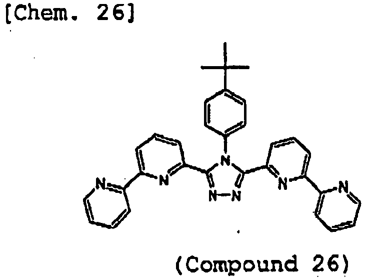

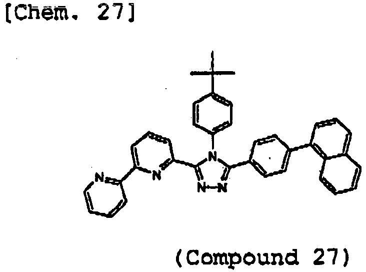

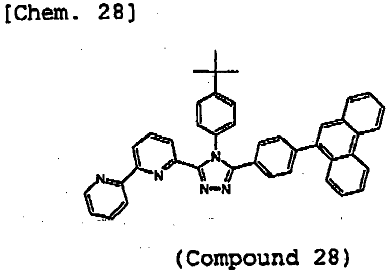

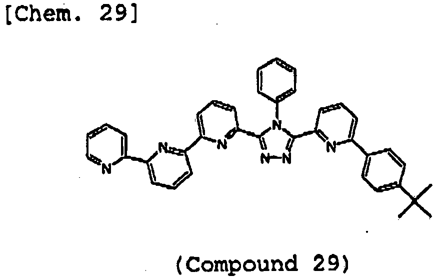

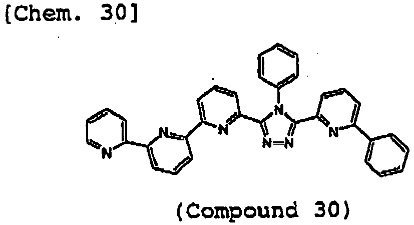

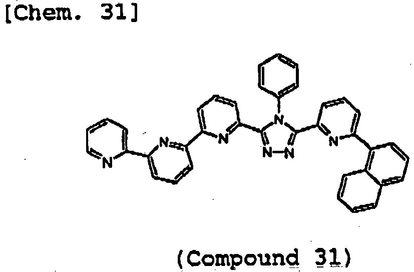

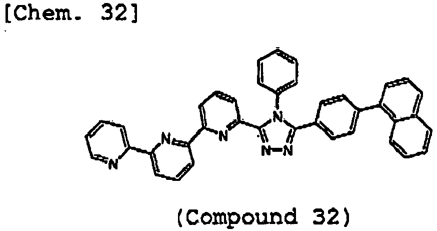

Purification of these compounds was carried out by column chromatography purification, adsorption purification, recrystallization using a solvent, a crystallization method, or the like. The compounds were identified by NMR analysis. For physical property values, DSC measurement (Tg) and melting point measurement were performed. The melting point serves as an index of vapor deposition property, and the glass transition point (Tg) serves as an index of stability in a thin film state.

The melting point and the glass transition point were measured by using a powder and by using DSC6220 which is a differential scanning calorimeter produced by Seiko Instruments Inc.

Work function was measured by preparing a thin film of 100 nm on an ITO substrate and by using AC3 which is an atmospheric photoelectron spectrometer produced by Riken Keiki Co., Ltd. The work function serves as an index of hole blocking property.

Examples of the structure of the organic EL device of this invention include a structure wherein an anode, hole injection layer, a hole transport layer, a luminescent layer, a hole blocking layer, an electron transport layer, an electron injection layer, and a cathode are formed in this order on a substrate. In the multi-layer structure, it is possible to omit an organic layer(s) by giving functions of plural layers to an organic layer in combination. For example, the device may have a structure wherein an anode, a hole injection layer-cum-transport layer, a luminescent layer, a hole blocking layer-cum-electron transport layer, an electron injection layer, and a cathode are formed in this order on a substrate.

As the anode of the organic EL device, an electrode material having a large work function such as ITO and gold may be used. As the hole injection layer, materials such as copper phthalocyanine (hereinafter abbreviated as CuPc), a triphenylamine derivative of starburst type, and a tris [p-(N-phenyl-α-naphthylamino)phenyl]amine as well as coating type materials may be used.

As the hole transport layer, benzidine derivatives such as N,N'-diphenyl-N,N'-di(m-tolyl)benzidine (hereinafter abbreviated as TPD) and N,N'-diphenyl-N,N'-di(α-naphthyl)benzidine (hereinafter abbreviated as NPD); various triphenylamine tetramers; and the like may be used. Also, as the hole injection layer-cum-hole transport layer, a coating type high molecular material such as poly(ethylenedioxythiophene)/poly(styrenesulfonate) (hereinafter abbreviated as PEDOT/PSS) may be used.

Also, it is possible to form the organic EL device by using poly-N-vinylcarbazole (hereinafter abbreviated as PVK) as a hole injection layer-cum-hole transport layer-cum-luminescent layer as disclosed in Japanese Patent No. 2734341 and combining it with a layer of the compound of this invention.

As the luminescent layer, the hole blocking layer, the electron transport layer of the organic EL device of this invention, an aluminum complex, an oxazole derivative, a carbazole derivative, a phenanthroline derivative, a polydialkyl fluorene derivative, and the like may be used as well as the compound having a triazole ring structure to which a substituted pyridyl group is bonded.

By using the conventional luminescent material such as an aluminum complex and a styryl derivative for the luminescent layer and using the compound having a triazole ring structure to which a substituted pyridyl group is bonded as the hole blocking layer, the electron transport layer, or the hole blocking layer-cum-electron transport layer, it is possible to produce a high performance organic EL device. Also, it is possible to produce a high performance organic EL device by adding a dopant which is a fluorescent material such as quinacridone, coumarin and rubrene, or a phosphorescent material such as an iridium complex of phenylpyridine.

Further, a layer obtained by overlaying or codepositing a conventional electron transport material and an electron injection material on the compound having a triazole ring structure to which a substituted pyridyl group is bonded may be used as the electron transport layer.

As the electron injection layer, lithium fluoride, cerium, and the like may be used, but this layer may be omitted. As the cathode, an electrode material having a low work function such as aluminum or an alloy having a further low work function such as aluminum-magnesium may be used.

Embodiments of the present invention will be illustrated in greater detail with reference to the following Examples, but the invention should not be construed as being limited thereto so long as not exceeding the gist thereof.

### EXAMPLE 1

### (Synthesis of 3-(2,2'-bipyridine-6-yl)-4-phenyl-5-(6-bromopyridine-2-yl)-1,2,4-triazole)

A reaction vessel substituted with nitrogen was charged with 4.3 ml of aniline and 80 ml of 1,2-dichlorobenzene, and 0.75 ml of phosphorus trichloride was added thereto at a room temperature, followed by stirring at 100°C for one hour. After cooling to 50°C or less, 3.1 g of N-(2,2'-bipyridine-6-carbonyl)-N'-(6-bromo-2-pyridinylcarbonyl)hydrazide was added to allow a reaction to proceed at 140°C to 150°C for 5 hours. After cooling the reaction solution to 50°C, water was added thereto followed by stirring for one hour. After that, the reaction solution was extracted by chloroform, and an organic layer was dried by sodium carbonate. After sodium carbonate was removed by filtration, the filtrate was concentrated under a reduced pressure, and the thus-obtained solid was purified by column chromatography (carrier: NH silica gel; eluent: chloroform/hexane = 7/3), thereby obtaining 1.0 g of 3-(2,2'-bipyridine-6-yl)-4-phenyl-5-(6-bromopyridine-2-yl)-1,2,4-triazole (yield: 28%).

### (Synthesis of 3-(2,2'-bipyridine-6-yl)-4-phenyl-5-[6-(4-tert-butylphenyl)pyridine-2-yl]-1,2,4-triazole (hereinafter abbreviated as BpyTAZ01)(Compound 2))

A reaction vessel substituted with argon was charged with 3.0 g of 3-(2,2'-bipyridine-6-yl)-4-phenyl-5-(6-bromopyridine-2-yl)-1,2,4-triazole and 1.75 g of 4-tert-butylphenylboronic acid, and 100 ml of a degassed solution of toluene/ethanol (4/1, v/v) and 20.0 ml of a 1M-potassium carbonate solution were added thereto, followed by adding 0.40 g of tetrakis(triphenylphosphine)palladium (0) under the argon atmosphere. A reaction was allowed to proceed for 3 hours under reflux, and then the reaction solution was cooled to a room temperature, followed by filtration of a precipitated solid. The residue was dissolved into chloroform, and the solution was dehydrated by anhydrous magnesium sulfate, followed by filtration. After condensing the filtrate, crystallization purification by chloroform/n-hexane was performed twice, thereby obtaining 2.70 g of BpyTAZ01 (Compound 2) as a white powder (yield:' 81%).

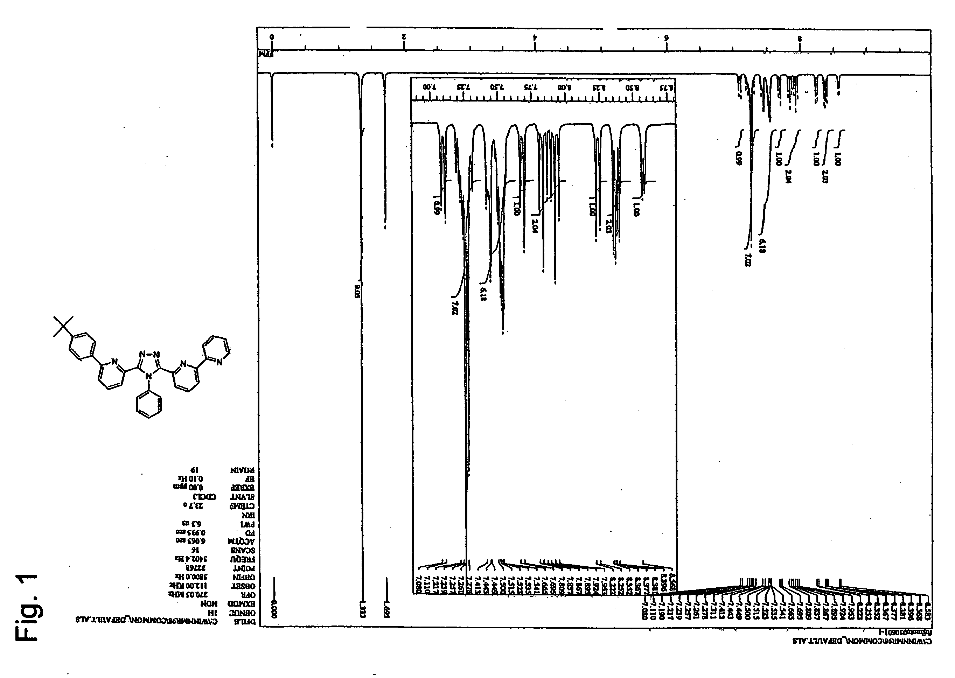

A structure of the thus-obtained powder was identified by using NMR. 1H-NMR measurement results are shown in Fig. 1.

The following 28 hydrogen signals were detected by 1H-NMR (CDCl₃). δ(ppm) = 8.576 ppm (1H), 8.396-8.352 ppm (2H), 8.238 ppm (1H), 7.953-7.809 ppm (2H), 7.680 ppm (1H), 7.535-7.413 ppm (6H), 7.278-7.217 (5H), 7.095 ppm (1H), 1.333 ppm (9H).

### EXAMPLE 2

### (Synthesis of 3-(2,2'-bipyridine-6-yl)-4-phenyl-5-[6-(1-naphthyl)pyridine-2-yl]-1,2,4-triazole (hereinafter abbreviated as BpyTAZ02)(Compound 9))

A reaction vessel substituted with argon was charged with 3.0 g of 3-(2,2'-bipyridine-6-yl)-4-phenyl-5-(6-bromopyridine-2-yl)-1,2,4-triazole, 1.70 g of 1-naphthaleneboronic acid, and 100 ml of a degassed solution of toluene/ethanol (4/1, v/v) and 20.0 ml of a 1M-potassium carbonate solution were added thereto, followed by adding 0.38 g of tetrakis(triphenylphosphine)palladium (0) under the argon atmosphere. A reaction was allowed to proceed for 2 hours under reflux, and then the reaction solution was cooled to a room temperature, followed by adding 100 ml of toluene. An organic layer and a water layer were separated from each other to remove the water layer. The organic layer was washed with a saturated sodium hydrogen carbonate solution and then dehydrated by anhydrous magnesium sulfate, followed by filtration. A solid precipitated by concentrating the filtrate was dissolved into toluene, and then 10 g of silica gel was added thereto for adsorption, followed by filtration for removing the silica gel. The filtrate after the adsorption was concentrated, and crystallization purification of the thus-obtained solid was performed by chloroform/n-hexane, followed by filtration. Methanol was added to the thus-collected solid for washing under reflux for one hour, followed by cooling to a room temperature and filtration, thereby obtaining 2.12 g of BpyTAZ02 (Compound 9) as a white powder (yield: 64%).

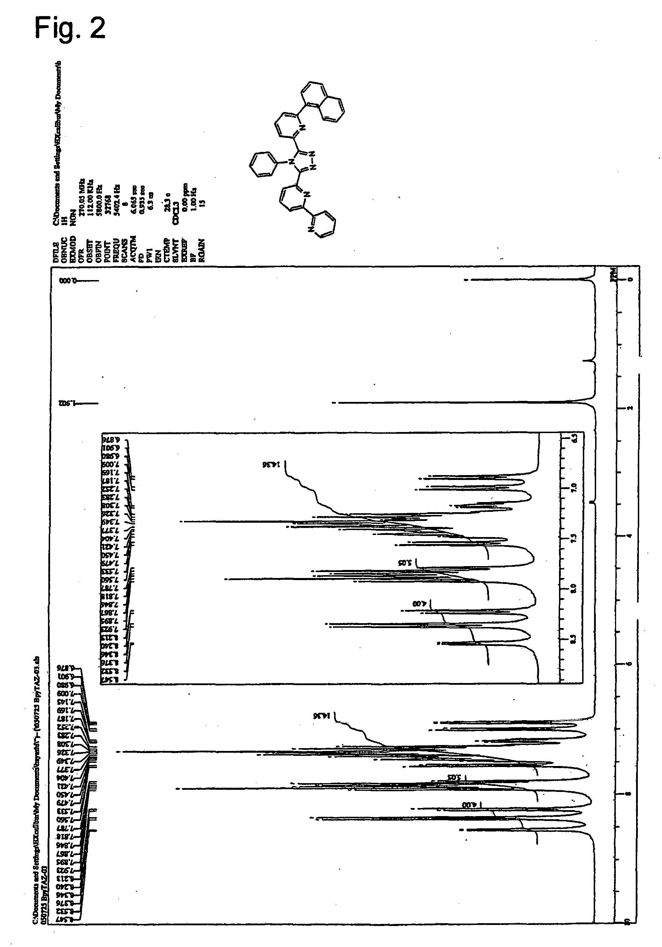

A structure of the thus-obtained powder was identified by using NMR. 1H-NMR measurement results are shown in Fig. 2.

The following 22 hydrogen signals were detected by 1H-NMR (CDCl₃). δ(ppm) = 8.540 ppm (1H), 8.376-8.346 ppm (2H), 8.227 ppm (1H), 7.923-7.787 ppm (5H), 7.560-7.169 ppm (11H), 6.995 ppm (1H), 6.889 ppm (1H).

### EXAMPLE 3

### (Synthesis of 3-(2,2'-bipyridine-6-yl)-4-phenyl-5-[6-(phenanthrene-9-yl)pyridine-2-yl]-1,2,4-triazole (Compound 11))

To a reaction vessel substituted with argon, 1.35 g of 3-(2,2'-bipyridine-6-yl)-4-phenyl-5-(6-bromopyridine-2-yl)-1,2,4-triazole, 1.0 g of 9-phenanthreneboronic acid, 50 ml of a degassed solution of toluene/ethanol (4/1, v/v), and 8.9 ml of a 1 M-potassium carbonate solution were added, followed by adding thereto 0.17 g of tetrakis(triphenylphosphine)palladium (0) under the argon atmosphere. A reaction was allowed to proceed for 9 hours under reflux, and then 0.09 g of tetrakis(triphenylphosphine)palladium (0) was added to allow the reaction to proceed for 3.5 hours more under reflux. After completion of the reaction, the reaction solution was cooled to a room temperature, and a precipitated crude product was collected by filtration. To the thus-obtained crude product, 100 ml of chloroform was added, followed by elimination of the catalyst by filtration. After concentrating the filtrate, crystallization purification was performed by adding methanol. The thus-obtained white solid was dried at 50°C overnight under a reduced pressure, thereby obtaining 1.36 g of 3-(2,2'-bipyridine-6-yl)-4-phenyl-5-[6-(phenanthrene-9-yl)pyridine-2-yl]-1,2,4-triazole (Compound 11) (yield: 83%).

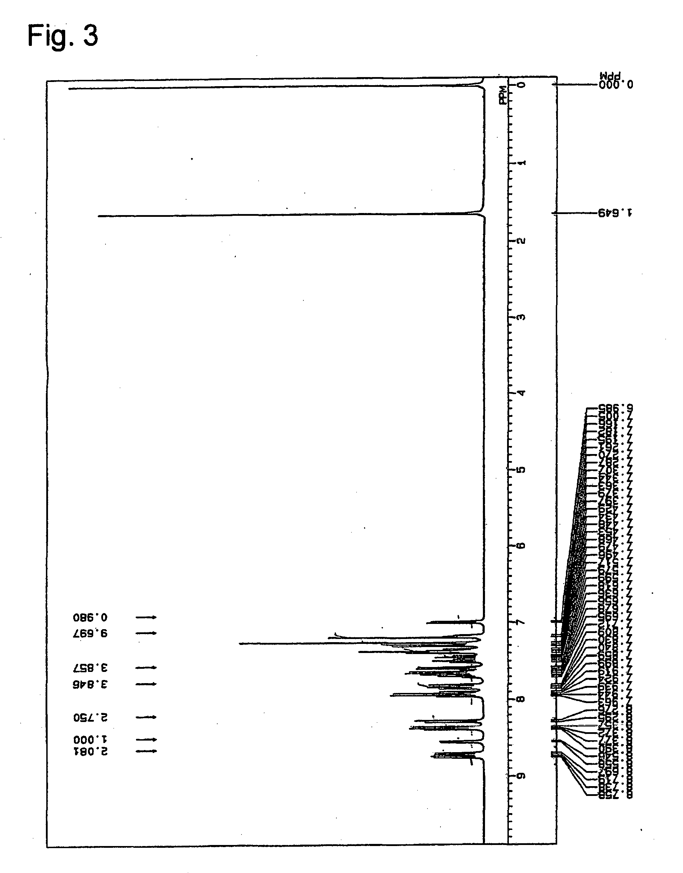

A structure of the thus-obtained white solid was identified by using NMR. 1H-NMR measurement results are shown in Fig. 3.

The following 24 hydrogen signals were detected by 1H-NMR (CDCl₃). δ(ppm) = 8.758-8.697 ppm (2H), 8.552 ppm (1H) 8.390-8.357 ppm (2H), 8.286 ppm (1H), 7.963-7.899 ppm (2H), 7.859-7.809 ppm (2H), 7.712-7.579 ppm (4H), 7.517-7.166 ppm (9H), 6.995 ppm (1H).

### EXAMPLE 4

### (Synthesis of 3-(2,2'-bipyridine-6-yl)-4-phenyl-5-[6-(biphenyl-2-yl)pyridine-2-yl]-1,2,4-triazole (Compound 15))

To a reaction vessel substituted with argon, 2.0 g of 3-(2,2'-bipyridine-6-yl)-4-phenyl-5-(6-bromopyridine-2-yl)-1,2,4-triazole, 1.31 g of 2-biphenylboronic acid, 75 ml of a degassed solution of toluene/ethanol (4/1, v/v), and 13.24 ml of a 1M-potassium carbonate solution were added, followed by adding thereto 0.26 g of tetrakis(triphenylphosphine)palladium (0) under the argon atmosphere. A reaction was allowed to proceed for 6 hours under reflux, and, after completion of the reaction, the reaction solution was cooled to a room temperature, followed by adding 200 ml of ethyl acetate. An organic layer and a water layer were separated from each other to remove the water layer. The organic layer was washed with a saturated saline and then dehydrated by anhydrous magnesium sulfate, followed by concentration. The thus-obtained crude product was purified by recrystallization and dried at 50°C overnight under a reduced pressure, thereby obtaining 1.25 g of 3-(2,2'-bipyridine-6-yl)-4-phenyl-5-[6-(biphenyl-2-yl)pyridine-2-yl]-1,2,4-triazole (Compound 15) (yield: 54%).

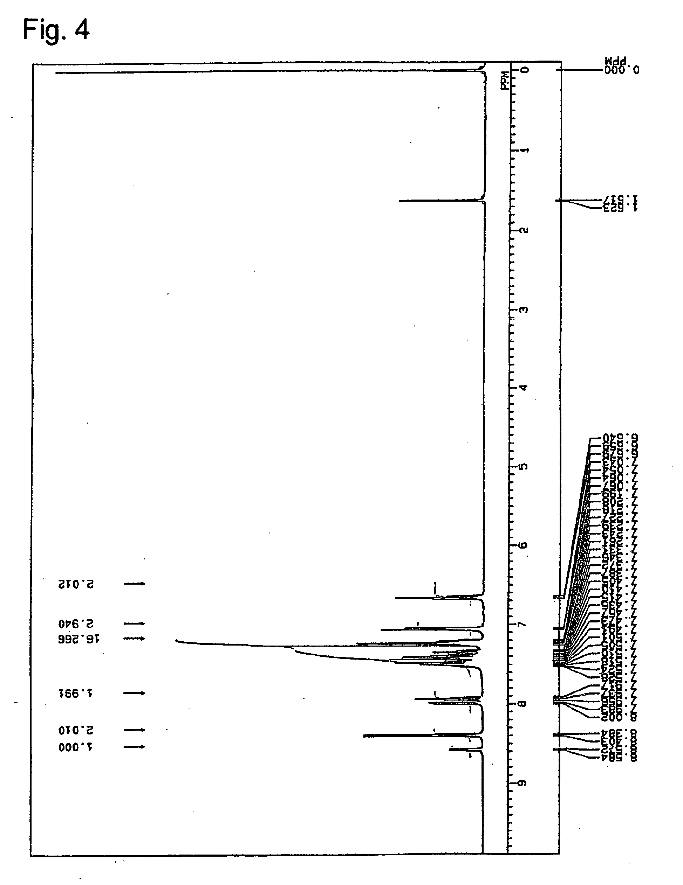

A structure of the thus-obtained white solid was identified by using NMR. 1H-NMR measurement results are shown in Fig. 4.

The following 24 hydrogen signals were detected by 1H-NMR (CDCl₃). δ(ppm) = 8.578 ppm (1H), 8.394 ppm (2H), 8.002-7.917 ppm (2H), 7.528-7.199 ppm (14H), 7.067-7.043 ppm (3H), 6.679-6.640 ppm (2H).

### EXAMPLE 5

### (Synthesis of 3-(2,2'-bipyridine-6-yl)-4-phenyl-5-[6-(9,9-dimethylfluorene-2-yl)pyridine-2-yl]-1,2,4-triazole (Compound 16))

To a reaction vessel substituted with argon, 1.0 g of 3-(2,2'-bipyridine-6-yl)-4-phenyl-5-(6-bromopyridine-2-yl)-1,2,4-triazole, 0.85 g of 2-(9,9-dimethylfluorene-2-yl)-4,4,5,5-tetramethyl-[1,3,2]dioxaborate, 40 ml of a degassed solution of toluene/ethanol (4/1, v/v), and 5.5 ml of a 2M-potassium carbonate solution were added, followed by adding thereto 0.13 g of tetrakis(triphenylphosphine)palladium (0) under the argon atmosphere. A reaction was allowed to proceed for 7.5 hours under reflux. After completion of the reaction, the reaction solution was cooled to a room temperature and concentrated. After adding 200 ml of chloroform, an organic layer and a water layer were separated from each other to remove the water layer. The organic layer was washed with a saturated saline and then dehydrated by anhydrous magnesium sulfate, followed by concentration. After dissolving the thus-obtained crude product into chloroform, 20 g of NH silica gel was added to perform adsorption purification, and then crystallization purification was performed by chloroform/methanol, followed by drying overnight under a reduced pressure, thereby obtaining 1.14 g of 3-(2,2'-bipyridine-6-yl)-4-phenyl-5-[6-(9,9-dimethylfluorene-2-yl)pyridine-2-yl]-1,2,4-triazole (Compound 16) (yield: 91%).

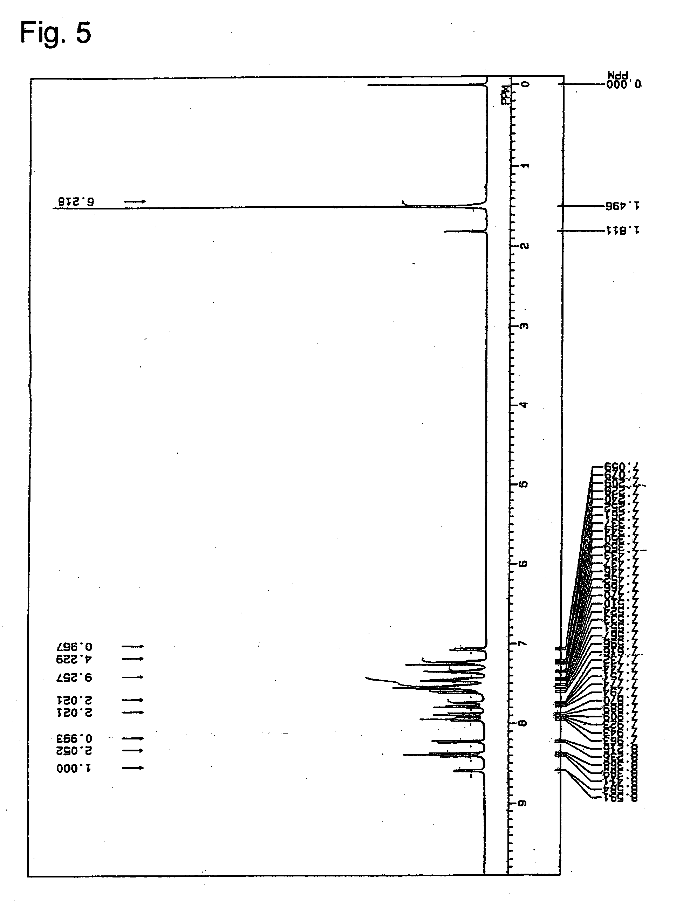

A structure of the thus-obtained white solid was identified by using NMR. 1H-NMR measurement results are shown in Fig. 5.

The following 28 hydrogen signals were detected by 1H-NMR (CDCl₃). δ(ppm) = 8.589 ppm (1H),8.411-8.368 ppm (2H), 8.266 ppm (1H), 7.963-7.870 ppm (2H), 7.794-7.732 ppm (2H), 7.616-7.433 ppm (9H), 7.359-7.209 ppm (4H), 7.069 ppm (1H), 1.496 ppm (6H).

### EXAMPLE 6

Melting points and glass transition points of the compounds of this invention were detected by a high sensitivity differential scanning calorimeter (DSC3100S; product of Bruker AXS K.K.).

| | Melting Point | Glass Transition Point |
|---|---|---|
| Compound of Ex. 1 of this invention | 268°C | - |
| | | |
| Compound of Ex. 2 of this invention | 205°C | 74°C |
| | | |
| Compound of Ex. 3 of this invention | 256°C | 96°C |
| | | |
| Compound of Ex. 4 of this invention | 230°C | 79°C |
| | | |
| Compound of Ex. 5 of this invention | 311°C | 104°C |

### EXAMPLE 7

A vapor deposition film having a film thickness of 100 nm was formed on an ITO substrate by using each of the compounds of this invention, and work functions thereof were measured by atmospheric photoelectron spectrometer (AC3; product of Riken Keiki Co., Ltd.).

| | Work Function |
|---|---|
| Compound of Ex. 1 of this invention | 6.30 eV |
| Compound of Ex. 2 of this invention | 6.30 eV |
| Compound of Ex. 3 of this invention | 6.27 eV |
| Compound of Ex. 4 of this invention | 6.28 eV |
| Compound of Ex. 5 of this invention | 6.06 eV |

The compounds each having a triazole ring structure to which a substituted pyridyl is bonded have the work functions that are prominently larger than TAZ which has the work function of 5.8 eV, and it is apparent that the compounds have excellent hole blocking property.

### EXAMPLE 8

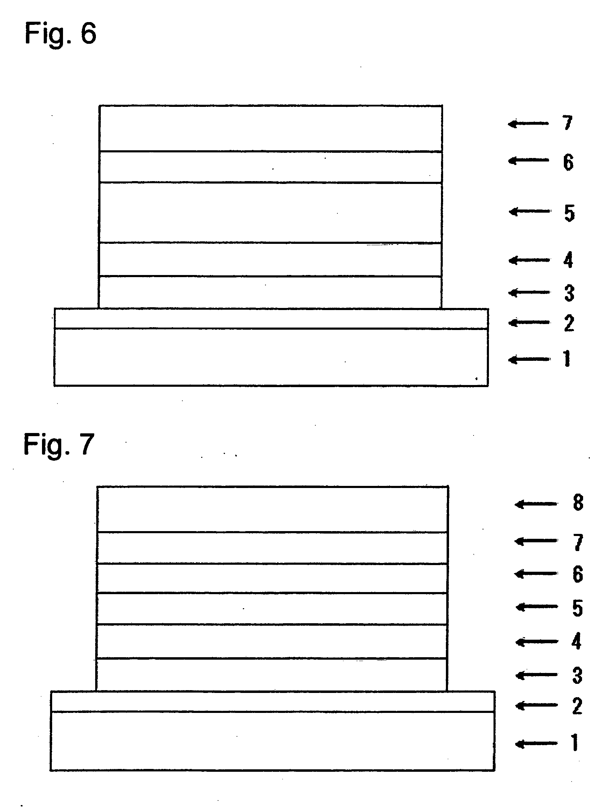

As shown in Fig. 6, an organic EL device was produced by stacking, on a glass substrate 1 on which an ITO electrode had been formed as a transparent anode 2, a hole injection layer-cum-hole transport layer 3, a luminescent layer 4, a hole blocking layer-cum-electron transport layer 5, an electron injection layer 6, and a cathode (aluminum electrode) 7 in this order.

The glass substrate 1 on which an ITO film having a film thickness of 150 nm had been formed was washed with an organic solvent and then subjected to washing of its surface by an oxygen plasma treatment.

On the ITO substrate, about 50 nm of NPD was vapor-deposited at a vapor deposition rate of 6 nm/min by using a vacuum vapor deposition device to form the hole injection layer-cum-hole transport layer 3. Subsequently, about 20 nm of Alq was formed as the luminescent layer 4 at a vapor deposition rate of 6 nm/min. On the luminescent layer 4, about 30 nm of BpyTAZ01 (Compound 2) which is the compound of Example 1 of this invention was formed as the hole blocking layer-cum-electron transport layer 5 at a vapor deposition rate of 6 nm/min. Further, about 0.5 nm of lithium fluoride was formed as the electron injection layer 6 at a vapor deposition rate of 0.1 angstrom/s. Lastly, with inserting a mask for cathode vapor deposition, about 200 nm of aluminum was vapor-deposited to form the cathode 7. The thus-obtained device was stored in a vacuum desiccator, and characteristic measurement was performed in the air at an ordinary temperature.

The thus-obtained organic EL device generated stable green luminescence and exhibited luminescence of 2,900 cd/m² · at a driving voltage of 6.0 V and luminescence of 5,100 cd/m² at a driving voltage of 7.0 V.

A ratio obtained by dividing the number of photons emitted from the organic EL device by the number of electrons injected into the organic EL device is called an external quantum efficiency. The external quantum efficiencies of this device at 10,000 cd/m² and 20,000 cd/m² were 1.29% and 1.46%, respectively.

A maximum luminance before breakpoint was evaluated by increasing the load of the current density by increasing the driving voltage. Since the maximum luminance measured by the method reflects electrical stability of the device, the maximum luminance serves as an index of durability of the organic EL device.

The organic EL device exhibited a maximum luminance of 28,500 cd/m² at 9.0 V, and then luminance of luminescence was diminished to reach the breakpoint.

### Example 9

An organic EL device was produced under the same conditions as in Example 8 except for using BpyTAZ02 which is the compound of Example 2 of this invention as the material for the hole blocking layer-cum-electron transport layer 5, and the characteristics thereof were examined. About 30 nm of BpyTAZ02 was formed at a vapor deposition rate of 6 nm/min as the hole blocking layer-cum-electron transport layer 5. The thus-obtained organic EL device exhibited luminescence of 3,700 cd/m² at a driving voltage of 6.0 V and luminescence of 13,000 cd/m² at a driving voltage of 7.0 V. The external quantum efficiencies of this device at 10,000 cd/m² and 20,000 cd/m² were 1.46% and 1.60%, respectively.

### [Comparative Example 1]

For comparison, an organic EL device was produced under the same conditions as in Example 8 except for replacing the material for the hole blocking layer-cum-electron transport layer 5 with TAZ, and the characteristics thereof were examined. About 30 nm of TAZ was formed at a vapor deposition rate of 6 nm/min as the hole blocking layer-cum-electron transport layer 5. The thus-obtained organic EL device exhibited luminescence of 260 cd/m² at a driving voltage of 6.0 V and luminescence of 780 cd/m² at a driving voltage of 7.0 V. The external quantum efficiency immediately before the breakpoint and the maximum luminance of the device were 0.95% and 9,500 cd/m².

### [Comparative Example 2]

For further comparison, an organic EL device was produced under the same conditions as in Example 8 except for replacing the material for the hole blocking layer-cum-electron transport layer 5 with Alq, and the characteristics thereof were examined. About 50 nm of Alq was formed at a vapor deposition rate of 6 nm/min as the luminescent layer-cum-hole blocking layer-cum-electron transport layer 4 and 5. The thus-obtained organic EL device exhibited luminescence of 850 cd/m² at a driving voltage of 6.0 V and luminescence of 2,800 cd/m² at a driving voltage of 7.0 V. The external quantum efficiencies of the device at 10,000 cd/m², 20,000 cd/m², and 30,000 cd/m² were 1.27%, 1.24%, and 0.97%, respectively. The maximum luminance immediately before the breakpoint was 31,600 cd/m².

As is apparent from the above results, the organic EL devices obtained by using the compound having a triazole ring structure to which a substituted pyridyl group is bonded prominently reduce a driving voltage of organic EL device as compared to the device obtained by using Alq which is used as an ordinary electron transport material and the device obtained by using TAZ which is an electron transport material excellent in hole blocking property. Further, it was confirmed that the organic EL devices are excellent also in luminescent efficiency.

From the prominent reduction in driving voltage in the above comparative experiments, it is expected that the electron mobility of the compounds having a triazole ring structure to which a substituted pyridyl group is bonded is higher than Alq which is an ordinary electron transport material, and considerably higher than TAZ which is a conventional hole blocking material.

### EXAMPLE 10

An organic EL device was produced by stacking on a glass substrate 1 on which an ITO electrode had been formed as a transparent anode 2, a hole injection layer-cum-hole transport layer 3, a luminescent layer 4, a hole blocking layer 5, an electron transport layer 6, an electron injection layer 7, and a cathode (aluminum electrode) 8 in this order as shown in Fig. 7.

The glass substrate 1 on which an ITO film having a film thickness of 150 nm had been formed was washed with an organic solvent and then subjected to washing of its surface by an oxygen plasma treatment.

On the ITO substrate, about 50 nm of NPD was vapor-deposited at a vapor deposition rate of 6 nm/min by using a vacuum vapor deposition device to form the hole injection layer-cum-hole transport layer 3. Subsequently, about 20 nm of Alq was formed as the luminescent layer 4 at a vapor deposition rate of 6 nm/min. On the luminescent layer 4, about 20 nm of BpyTAZ01 (Compound 2) which is the compound of Example 1 of this invention was formed as the hole blocking layer 5 at a vapor deposition rate of 6 nm/min. On the hole blocking layer 5, about 10 nm of Alq was formed as the electron transport layer 6 at a vapor deposition rate of 6 nm/min. Further, about 0.5 nm of lithium fluoride was formed as the electron injection layer 7 at a vapor deposition rate of 0.1 angstrom/s. Lastly, with inserting a mask for cathode vapor deposition, about 200 nm of aluminum was vapor-deposited to form the cathode 8. The thus-obtained device was stored in a vacuum desiccator, and characteristic measurement was performed in the air at an ordinary temperature.

The external quantum efficiencies of the thus-obtained organic EL device at 10,000 cd/m², 20,000 cd/m², and 30,000 cd/m² were 1.13%, 1.46%, and 1.54%, respectively. The maximum luminance of luminescence immediately before the breakpoint was 31,200 cd/m².

### [Comparative Example 3]

For comparison, an organic EL device was produced under the same conditions as in Example 10 except for replacing the material for the hole blocking layer 5 with TAZ, and the characteristics thereof were examined. About 20 nm of TAZ was formed as the hole blocking layer 5 at a vapor deposition rate of 6 nm/min. The thus-obtained organic EL device reached the breakpoint at a maximum luminance of 12,000 cd/m².

As is apparent from the above results, it is possible to produce an organic EL device having good efficiency by using the compound having a triazole ring structure to which a substituted pyridyl group is bonded as a material for a hole blocking layer in combination with another electron transport material.

While the present invention has been described in detail and with reference to specific embodiments thereof, it will be apparent to one skilled in the art that various changes and modifications can be made therein without departing from the spirit and scope thereof.

This invention is based on the Japanese.patent application No. 2005-250734 filed on August 31, 2005, and the contents thereof are herein incorporated by reference.

### INDUSTRIAL APPLICABILITY

The compound of this invention having a triazole ring structure to which a substituted pyridyl group is bonded is excellent as a compound for organic EL devices since it is excellent in hole blocking property and has a high electron mobility. By producing an organic EL device using the compound, it is possible to dramatically reduce a driving voltage and to realize a device having high efficiency and durability. For example, expansions into utilization for home electric appliances and lighting have been made possible.

## Claims

1. A compound having a triazole ring structure to which a substituted pyridyl group is bonded, represented by the general formula (1): 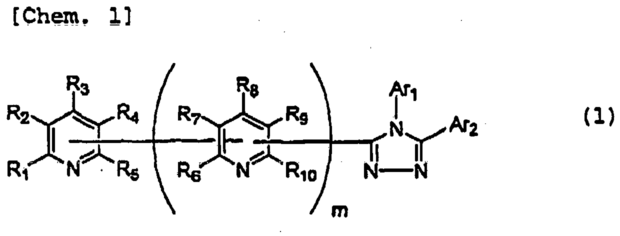 wherein Ar₁ and Ar₂ may be the same or different and each represents a substituted or unsubstituted aromatic hydrocarbon group, a substituted or unsubstituted aromatic heterocyclic group, or a substituted or unsubstituted condensed polycyclic aromatic group; any one of R₁, R₂, R₃, R₄, and R₅ is a bonding group and the rest thereof may be the same or different and each represents a hydrogen atom, a fluorine atom, a cyano group, an alkyl group, a substituted or unsubstituted aromatic hydrocarbon group, a substituted or unsubstituted aromatic heterocyclic group, or a substituted or unsubstituted condensed polycyclic aromatic group; any two of R₆, R₇, R₈, R₉, and R₁₀ are bonding groups and the rest thereof may be the same or different and each represents a hydrogen atom, a fluorine atom, a cyano group, an alkyl group, a substituted or unsubstituted aromatic hydrocarbon group, a substituted or unsubstituted aromatic heterocyclic group, or a substituted or unsubstituted condensed polycyclic aromatic group; and m represents an integer of 1 to 4.

2. The compound having the triazole ring structure according to claim 1, wherein m = 1 in the general formula (1).

3. The compound having the triazole ring structure according to claim 1, wherein m = 2 in the general formula (1).

4. An organic electroluminescence device comprising a pair of electrodes and at least one organic layer sandwiched therebetween, wherein the device comprises as a constituting material of the at least one organic layer a compound according to claim 1 having a triazole ring structure to which a substituted pyridyl group is bonded, represented by the following general formula (1): 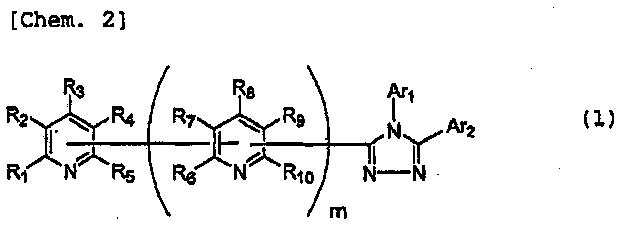 wherein Ar₁ and Ar₂ may be the same or different and each represents a substituted or unsubstituted aromatic hydrocarbon group, a substituted or unsubstituted aromatic heterocyclic group, or a substituted or unsubstituted condensed polycyclic aromatic group; any one of R₁, R₂, R₃, R₄, and R₅ is a bonding group and the rest thereof may be the same or different and each represents a hydrogen atom, a fluorine atom, a cyano group, an alkyl group, a substituted or unsubstituted aromatic hydrocarbon group, a substituted or unsubstituted aromatic heterocyclic group, or a substituted or unsubstituted condensed polycyclic aromatic group; any two of R₆, R₇, R₈, R₉, and R₁₀ are bonding groups and the rest thereof may be the same or different and each represents a hydrogen atom, a fluorine atom, a cyano group, an alkyl group, a substituted or unsubstituted aromatic hydrocarbon group, a substituted or unsubstituted aromatic heterocyclic group, or a substituted or unsubstituted condensed polycyclic aromatic group; and m represents an integer of 1 to 4.

5. The organic electroluminescence device according to claim 4, wherein m = 1 in the general formula (1).

6. The organic electroluminescence device according to claim 4, wherein m = 2 in the general formula (1).

7. The organic electroluminescence device according to claim 4, wherein the organic layer containing the compound represented by the general formula (1) is an electron transport layer.

8. The organic electroluminescence device according to claim 4, wherein the organic layer containing the compound represented by the general formula (1) is a hole blocking layer.

9. The organic electroluminescence device according to claim 4, wherein the organic layer containing the compound represented by the general formula (1) is a luminescent layer.

## Patentansprüche

1. Verbindung mit einer Triazolringstruktur, an die eine substituierte Pyridylgruppe gebunden ist, dargestellt durch die allgemeine Formel (1): 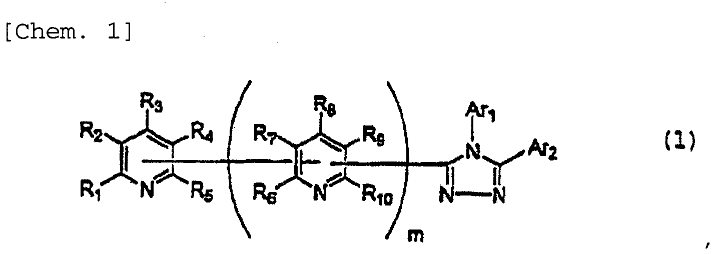 wobei Ar₁ und Ar₂ gleich oder verschieden sein können und jedes eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; eines von R₁, R₂, R₃, R₄ und R₅ eine Bindungsgruppe ist und der Rest gleich oder verschieden sein kann und jedes ein Wasserstoffatom, ein Fluoratom, eine Cyanogruppe, eine Alkylgruppe, eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; je zwei von R₆, R₇, R₈, R₉ und R₁₀ Bindungsgruppen sind und der Rest gleich oder verschieden sein kann und jedes ein Wasserstoffatom, ein Fluoratom, eine Cyanogruppe, eine Alkylgruppe, eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; und m eine ganze Zahl von 1 bis 4 darstellt.

2. Verbindung mit der Triazolringstruktur gemäß Anspruch 1, wobei in der allgemeinen Formel (1) m = 1 ist.

3. Verbindung mit der Triazolringstruktur gemäß Anspruch 1, wobei in der allgemeinen Formel (1) m = 2 ist.

4. Organische Elektrolumineszenzvorrichtung, die ein Elektrodenpaar und mindestens eine organische Schicht, die dazwischen eingeschoben ist, umfasst, wobei die Vorrichtung als Material der mindestens einen organischen Schicht eine Verbindung gemäß Anspruch 1 mit einer Triazolringstruktur, an die eine substituierte Pyridylgruppe gebunden ist, umfasst, wobei die Verbindung durch die folgende allgemeine Formel (1) dargestellt ist: 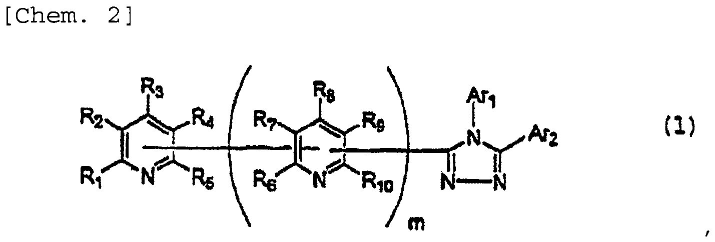 wobei Ar₁ und Ar₂ gleich oder verschieden sein können und jedes eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; eines von R₁, R₂, R₃, R₄ und R₅ eine Bindungsgruppe ist und der Rest gleich oder verschieden sein kann und jedes ein Wasserstoffatom, ein Fluoratom, eine Cyanogruppe, eine Alkylgruppe, eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; je zwei von R₆, R₇, R₈, R₉ und R₁₀ Bindungsgruppen sind und der Rest gleich oder verschieden sein kann und jedes ein Wasserstoffatom, ein Fluoratom, eine Cyanogruppe, eine Alkylgruppe, eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; und m eine ganze Zahl von 1 bis 4 darstellt.

5. Organische Elektrolumineszenzvorrichtung gemäß Anspruch 4, wobei in der allgemeinen Formel (1) m = 1 ist.

6. Organische Elektrolumineszenzvorrichtung gemäß Anspruch 4, wobei in der allgemeinen Formel (1) m = 2 ist.

7. Organische Elektrolumineszenzvorrichtung gemäß Anspruch 4, wobei die organische Schicht, welche die durch die allgemeinen Formel (1) dargestellte Verbindung enthält, eine Elektronentransportschicht ist.

8. Organische Elektrolumineszenzvorrichtung gemäß Anspruch 4, wobei die organische Schicht, welche die durch die allgemeine Formel (1) dargestellte Verbindung enthält, eine Lochsperrschicht ist.

9. Organische Elektrolumineszenzvorrichtung gemäß Anspruch 4, wobei die organische Schicht, welche die durch die allgemeine Formel (1) dargestellte Verbindung enthält, eine lumineszierende Schicht ist.

## Revendications

1. Composé présentant une structure de cycle triazole à laquelle un groupe pyridyle substitué est lié, représenté par la formule générale (1) : 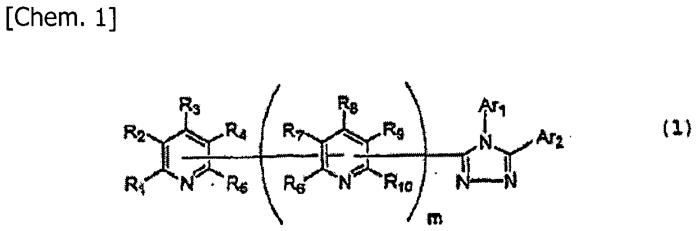 où Ar₁ et Ar₂ peuvent être identiques ou différents et représentent chacun un groupe hydrocarboné aromatique substitué ou non substitué, un groupe hétérocyclique aromatique substitué ou non substitué, ou un groupe aromatique polycyclique condensé substitué ou non substitué ; l'un quelconque de R₁, R₂, R₃, R₄, et R₅ est un groupe de liaison et les autres de ceux-ci peuvent être identiques ou différents et représentent chacun un atome d'hydrogène, un atome de fluor, un groupe cyano, un groupe alkyle, un groupe hydrocarboné aromatique substitué ou non substitué, un groupe hétérocyclique aromatique substitué ou non substitué, ou un groupe aromatique polycyclique condensé substitué ou non substitué ; deux quelconques de R₆, R₇, R₈, R₉, et R₁₀ sont des groupes de liaison et les autres de ceux-ci peuvent être identiques ou différents et représentent chacun un atome d'hydrogène, un atome de fluor, un groupe cyano, un groupe alkyle, un groupe hydrocarboné aromatique substitué ou non substitué, un groupe hétérocyclique aromatique substitué ou non substitué, ou un groupe aromatique polycyclique condensé substitué ou non substitué ; et m représente un nombre entier de 1 à 4.

2. Composé présentant la structure de cycle triazole selon la revendication 1, dans lequel m = 1 dans la formule générale (1).

3. Composé présentant la structure de cycle triazole selon la revendication 1, dans lequel m = 2 dans la formule générale (1).

4. Dispositif d'électroluminescence organique comprenant une paire d'électrodes et au moins une couche organique mise en sandwich entre celles-ci, dans lequel le dispositif comprend comme un matériau constituant de la au moins une couche organique un composé selon la revendication 1 présentant une structure de cycle triazole à laquelle un groupe pyridyle substitué est lié, représenté par la formule générale (1) suivante : 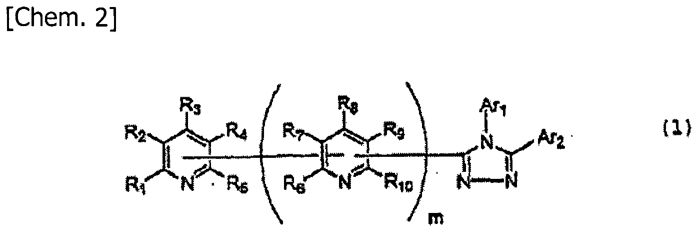 dans laquelle Ar₁ et Ar₂ peuvent être identiques ou différents et représentent chacun un groupe hydrocarboné aromatique substitué ou non substitué, un groupe hétérocyclique aromatique substitué ou non substitué, ou un groupe aromatique polycyclique condensé substitué ou non substitué ; l'un quelconque de R₁, R₂, R₃, R₄, et R₅ est un groupe de liaison et les autres de ceux-ci peuvent être identiques ou différents et représentent chacun un atome d'hydrogène, un atome de fluor, un groupe cyano, un groupe alkyle, un groupe hydrocarboné aromatique substitué ou non substitué, un groupe hétérocyclique aromatique substitué ou non substitué, ou un groupe aromatique polycyclique condensé substitué ou non substitué ; deux quelconques de R₆, R₇, R₈, R₉, et R₁₀ sont des groupes de liaison et les autres de ceux-ci peuvent être identiques ou différents et représentent chacun un atome d'hydrogène, un atome de fluor, un groupe cyano, un groupe alkyle, un groupe hydrocarboné aromatique substitué ou non substitué, un groupe hétérocyclique aromatique substitué ou non substitué, ou un groupe aromatique polycyclique condensé substitué ou non substitué ; et m représente un nombre entier de 1 à 4.

5. Dispositif d'électroluminescence organique selon la revendication 4, dans lequel m = 1 dans la formule générale (1).

6. Dispositif d'électroluminescence organique selon la revendication 4, dans lequel m = 2 dans la formule générale (1).

7. Dispositif d'électroluminescence organique selon la revendication 4, dans lequel la couche organique contenant le composé représenté par la formule générale (1) est une couche de transport d'électrons.

8. Dispositif d'électroluminescence organique selon la revendication 4, dans lequel la couche organique contenant le composé représenté par la formule générale (1) est une couche de blocage de trous.

9. Dispositif d'électroluminescence organique selon la revendication 4, dans lequel la couche organique contenant le composé représenté par la formule générale (1) est une couche luminescente.
